(19) Europäisches Patentamt
European Patent Office
Office européen des brevets

(11) Veröffentlichungsnummer : **0 142 070**
**B1**

(12) # EUROPÄISCHE PATENTSCHRIFT

(45) Veröffentlichungstag der Patentschrift : **09.07.86**

(51) Int. Cl.4 : **C 07 C 93/14**

(21) Anmeldenummer : **84112670.9**

(22) Anmeldetag : **19.10.84**

(54) **Verfahren zur Herstellung von N-Methylhomoveratrylamin.**

(30) Priorität : **25.10.83 DE 3338681**

(43) Veröffentlichungstag der Anmeldung : **22.05.85 Patentblatt 85/21**

(45) Bekanntmachung des Hinweises auf die Patenterteilung : **09.07.86 Patentblatt 86/28**

(84) Benannte Vertragsstaaten : **BE CH DE FR GB IT LI NL**

(56) Entgegenhaltungen :
**FR-A- 2 368 277**
**JOURNAL OF THE AMERICAN CHEMICAL SOCIETY, Band 72, Nr. 10, 16. Oktober 1950, Seiten 4364-4368, American Chemical Society, US; E. RONA: "Exchange reactions of uranium ions in solution"**
**CHEMICAL ABSTRACTS, Band 28, Nr. 1, 10. Januar 1934, Spalte 469, Nr. 5, Columbus, Ohio, US; K. KINDLER u.a.: "New and improved methods for the synthesis of pharmacologically important amines. VII. Synthesis of secondary and tertiary amines by the hydrogenation of nitriles"**

(73) Patentinhaber : **BASF Aktiengesellschaft**
**Carl-Bosch-Strasse 38**
**D-6700 Ludwigshafen (DE)**

(72) Erfinder : **Mueller, Josef, Dr.**
**Rieslingstrasse 15**
**D-6711 Grosskarlbach (DE)**
Erfinder : **Koernig, Wolfgang, Dr.**
**Dachsweg 5**
**D-6901 Dossenheim (DE)**
Erfinder : **Geiss, Karl-Heinz, Dr.**
**Kirchenstrasse 8**
**D-6711 Beindersheim (DE)**
Erfinder : **Wiersdorff, Walter-Wielant, Dr.**
**Blockfeldstrasse 15**
**D-6704 Mutterstadt (DE)**
Erfinder : **Baer, Karl, Dr.**
**Kastanienweg 1**
**D-6940 Weinheim (DE)**
Erfinder : **Lengsfeld, Wolfgang, Dr.**
**Woogstrasse 46**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein verbessertes Verfahren zur Herstellung von N-Methylhomoveratrylamin (MHVA) durch kontinuierliche oder diskontinuierliche Hydrierung von Veratrylcyanid (VCN) an einem Nickelkatalysator mit mindestens 70 Gew.% Nickel in Anwesenheit eines mindestens 10fach molaren Überschusses an Monomethylamin in Verbindung mit einer technisch leicht durchzuführenden Aufarbeitung.

MHVA wird als Pharmazwischenprodukt benötigt, insbesondere findet es Verwendung zur Herstellung des Wirkstoffs Verapamil (DE-PS 1 158 083).

Für die Herstellung sekundärer Amine sind aus der Literatur verschiedene Verfahren bekannt. Sie weisen alle mehr oder weniger Nachteile auf.

Beispielsweise beschreiben K. Kindler und F. Hesse in Archiv Pharmazie 271, 442 (1933) die Hydrierung von VCN zu MHVA in Anwesenheit von Methylamin und Palladium-Mohr als Katalysator. Es wurden jedoch nur 78 % Ausbeute erzielt, obwohl über 10 Gew.%, bezogen auf VCN, des sehr teuren Palladiumkatalysators eingesetzt wurden. Über die Wiederverwendbarkeit des Katalysators bei dieser diskontinuierlichen Verfahrensweise wurden keine Angaben gemacht.

Auch die Alkylierung der Schiffschen Base aus Homoveratrylamin (HVA) und Benzaldehyd mit Dimethylsulfat und anschließende Hydrolyse ist zur Synthese von MHVA vorgeschlagen worden (K. Kindler und W. Peschke, Archiv Pharmazie, 270, 340, 1932). Basierend auf VCN ist diese Verfahrensweise jedoch zweistufig und benötigt als Reagenz das hochgiftige Dimethylsulfat. Bei dieser Verfahrensweise bildet sich außerdem als Nebenprodukt N,N-Dimethylhomoveratrylamin, das technisch nur schwierig vom MHVA zu trennen ist.

In der DE-PS 1 493 754 wird die Hydrierung von Fettsäurenitrilen in Anwesenheit von Methylamin an einem Kupfer-Chrom-Kalium-Katalysator auf Kieselgel beschrieben. Hier werden zwar hohe Selektivitäten an gewünschtem sekundären Amin erreicht, doch sind Reaktionstemperaturen von etwa 150 °C erforderlich. Bei diesen Temperaturen können bei MHVA jedoch bereits Zersetzungen auftreten.

Der Erfindung lag daher die Aufgabe zugrunde, ein technisch einfach und risikolos durchzuführendes Verfahren zur Herstellung von MHVA mit hohen Ausbeuten zu entwickeln.

Die Lösung dieser Aufgabe besteht in dem Verfahren nach Anspruch 1. Die Umsetzung erfolgt nach folgender Gleichung :

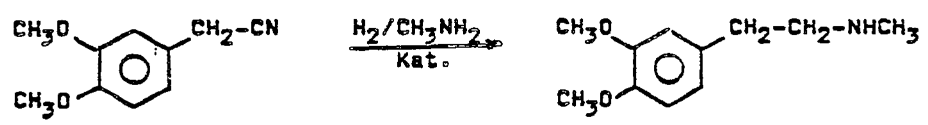


Veratrylcyanid (VCN) N-Methylhomoveratrylamin (MHVA)

Der Hydrierkatalysator ist ein nach üblichen Methoden, beispielsweise durch Fällen von Nickelcarbonat aus wäßriger Lösung und Glühen, hergestellter Nickelkatalysator mit einem Nickelgehalt von mindestens 70, vorzugsweise mindestens 80 Gew.%, bezogen auf den trägerlosen Katalysator. Ganz besonders bevorzugt sind Nickelgehalte von über 90 %. Der Nickelgehalt kann auch 100 % erreichen, doch wird ein Gehalt an bis zu 15, insbesondere von 3 bis 7 Gew.% Kobalt bevorzugt. Auch andere Metalle, beispielsweise Aluminium und Eisen, kommen als Mischungskomponenten in geringer Menge, und zwar insgesamt bis zu maximal 15, vorzugsweise bis zu 5 %, in Betracht. Der Katalysator ist zwar üblicherweise trägerlos, er kann aber auch auf eines der üblichen Trägermaterialien wie Kieselgel, Ton, Aluminiumoxid aufgebracht werden. Dies geschieht in üblicher Weise durch (ggf. wiederholtes) Tränken des Trägermaterials mit einer Nickelsalzlösung, Trocknen und Tempern bei ca. 400 bis 450 °C. Vor dem Einsatz wird der Katalysator in bekannter Weise drucklos oder unter Druck bei 250 bis 350 °C mit Wasserstoff behandelt.

Das Verfahren wird in der Weise durchgeführt, daß das VCN mit oder ohne Lösungsmittel in Gegenwart von überschüssigem Methylamin bei einem Wasserstoffdruck von 100 bis 300, vorzugsweise 180 bis 220 bar und 20 bis 140, vorzugsweise 50 bis 100 °C an dem oben beschriebenen Katalysator hydriert wird.

Als Lösungsmittel haben sich vor allem Alkohole und Amide oder deren Gemische bewährt, wobei bevorzugt Methanol und/oder N-Methylpyrrolidon zur Anwendung kommen. Dabei kann die Konzentration des eingesetzten VCN in dem jeweiligen Lösungsmittel bis zur Sättigungskonzentration unter den Hydrierbedingungen reichen. Im allgemeinen liegt sie bei 5 bis 50, vorzugsweise bei 10 bis 20 Gew.-%. Ein Lösungsmittel ist zwar nicht unbedingt erforderlich, weil bereits das überschüssige Methylamin, jedenfalls bei erhöhter Temperatur, als Lösungsmittel genügt. Trotzdem wird der Einsatz von Lösungsmitteln bevorzugt, weil sich das VCN als Lösung besser zudosieren läßt.

Das Verfahren wird bevorzugt kontinuierlich durchgeführt, wobei man VCN im Gemisch mit Methylamin im Molverhältnis 1 : 10 bis 1 : 100, vorzugsweise im Verhältnis 1 : 30 bis 1 : 50, hydriert. Nicht umgesetztes Methylamin kann, zweckmäßig nach Abtrennung des entstandenen Ammoniaks, ganz oder

teilweise in den Reaktor zurückgeführt werden. Der Durchsatz an VCN pro 100 l Katalysator und Stunde liegt im Bereich von 2 bis 20, vorzugsweise 4 bis 15, insbesondere 4 bis 8 kg.

Der Reaktionsraum besteht im einfachsten Fall aus einem druckfest verschlossenen Rohr mit Ein- und Auslaßventilen, das den Katalysator als Festbett enthält, und das stehend oder liegend angeordnet sein kann. Man kann aber auch einen Rührautoklaven mit entsprechenden Ventilen verwenden, wobei der Katalysator in der Lösung suspendiert ist.

Überraschenderweise erreicht man bei Verwendung des beschriebenen Nickelkatalysators ebenso wie bei dem Verfahren der eingangs genannten DE-PS 1 493 754 ausgezeichnete Selektivitäten an sekundärem Amin (MHVA), wobei die Reaktionstemperatur um bis zu etwa 100 °C niedriger liegt als beim genannten Verfahren. Gerade für die methylierende Hydrierung von VCN ist eine relativ niedrige Reaktionstemperatur von ganz entscheidender Bedeutung, da bei derartigen Verbindungen mit Phenylalkylethergruppen bei hoher thermischer Belastung und noch dazu im alkalischen Milieu stets mit einer Isomerisierung durch Alkylgruppenwanderung zu rechnen ist. Diese unerwünschte Nebenreaktion wird durch die niedrige Reaktionstemperatur im erfindungsgemäßen Verfahren vermieden. So sind tertiäre Amine im Hydrieraustrag praktisch nicht nachweisbar. Primäre Amine sind meist in Mengen von etwa 3 bis 8 % enthalten und werden anschließend, wie weiter unten näher beschrieben, abgetrennt. Der Rest ist das gewünschte sekundäre Amin (MHVA) in einer Ausbeute von rund 90 % der Theorie.

Um den hohen Reinheitsanforderungen für Pharmazwischenprodukte gerecht zu werden, das heißt einen Gehalt an primärem Homoveratrylamin von unter 0,5 % zu erreichen, wird der Hydrieraustrag, ggf. nach Entfernen des Lösungsmittels, mit einem Aldehyd, der in alpha-Stellung zur Aldehydgruppe keinen Wasserstoff trägt, vorzugsweise Benzaldehyd, bei Temperaturen von 20 bis 100 °C umgesetzt und anschließend das Reaktionsgemisch bei Temperaturen von 20 bis 150, vorzugsweise 20 bis 50 °C hydriert. Diese Hydrierung kann beispielsweise katalytisch mit Wasserstoff oder mit komplexen Hydriden erfolgen. Besonders bewährt hat sich hierfür eine wäßrige Natriumboranatlösung.

Üblicherweise orientieren sich die Mengen an Aldehyd und Reduktionsmittel am Gehalt an primärem Amin im ersten Hydrieraustrag. Im allgemeinen reichen äquivalente Mengen, bezogen auf das primäre Amin, aus, ein geringer Überschuß bis zu 5 % hat sich jedoch als vorteilhaft erwiesen.

Das so behandelte Rohamin wird einer Vakuumdestillation (ca. 1 bis 15 mbar) unterworfen, wobei das gewünschte sekundäre Amin praktisch frei von primären und tertiären Aminen erhalten wird. Eine Destillierkolonne ist dabei nicht erforderlich.

Der Vorteil des erfindungsgemäßen Verfahrens besteht darin, daß man im Gegensatz zu den bisher bekannten Verfahren auf schonende Weise aus VCN in einer Einstufenreaktion mit hohen Ausbeuten MHVA erhält, und zwar ohne Anwendung so hochgiftiger Stoffe wie Dimethylsulfat. Das MHVA läßt sich daraus in einfacher Weise in hochreiner Form isolieren.

Die in den Beispielen genannten Teile (Tle) und Prozente beziehen sich auf das Gewicht. Raumteile (RT) verhalten sich zu Gewichtsteilen wie Liter zu Kilogramm.

## Beispiel 1

### a) Herstellung des Katalysators

70 kg einer 20 %igen Sodalösung wurden auf 70 °C erhitzt. Zu der heißen Lösung wurde innerhalb von 100 min ein Gemisch von 49,45 kg einer 13,5 % Nickel enthaltenden Nickelnitratlösung mit 2,98 kg einer 11,8 % Kobalt enthaltenden Kobaltnitratlösung einfließen lassen. Dabei wurde ein pH von 7 erreicht. Bei diesem pH wurde 0,5 h nachgerührt, anschließend filtriert und 16 h auf einer Filterpresse gewaschen, bis im abfließenden Waschwasser eine Leitfähigkeit von unter 50 µs gemessen wurde.

Der Filterkuchen aus basischem Carbonat wurde anschließend bei 120 °C bis zur Gewichtskonstanz getrocknet. Man erhielt ein lockeres Pulver mit einem Litergewicht von 652 g und einem Glühverlust bei 900 °C von 30,5 Gew.-%. Das Pulver wurde bei 400 °C unter Überleiten von Luft in einem Drehrohrofen zersetzt.

Das so gewonnene Oxid wurde unter Zugabe von 2 % konzentrierter Salpetersäure in einem Kneter mit wenig Wasser verknetet, auf einer Strangpresse zu 4 mm starken Strängen verformt, getrocknet bei 120 °C und 1 h bei 450 °C getempert.

Die so hergestellten Strangpreßlinge wiesen ein Litergewicht von 2 320 g bei einer Porosität von 0,115 $cm^3/g$ auf. Röntgenografisch war etwas Spinell neben sehr viel Nickeloxid nachzuweisen.

### b) Hydrierung

Über 250 RT des wie oben beschrieben hergestellten Nickel-Kobalt-Katalysators ließ man bei einer Temperatur von 90 °C stündlich 100 RT (84,6 Tle) einer 20 %igen methanolischen VCN-Lösung und 162 Tle. Methylamin bei einem Druck von 200 bar in Gegenwart von Wasserstoff (ca. 120 000 RT pro Stunde drucklos gemessen) strömen. Das Reaktionsgemisch wurde über ein druckgeregeltes Austragsventil auf Normaldruck entspannt. Durch einfache Destillation entfernte man gelöste Gase und Methanol. Das zurückbleibende rohe Reaktionsgemisch hatte folgende (gaschromatographisch gemessene) Zusammensetzung :

| | |
|---|---|
| Methylhomoveratrylamin | 92,5 % |
| Homoveratrylamin | 3,3 % |
| Leichtsiedende Bestandteile | 2,8 % |
| Destillationsrückstand | 1,4 % |

c) Aufarbeitung

1 000 Tle. dieses Gemisches wurden in einem beheizbaren Rührgefäß mit 2 000 RT Fassungsvermögen vorgelegt und auf 50 °C vorgeheizt. Dann gab man langsam unter Kühlung, um die Temperatur zu halten, 29 Tle. Benzaldehyd zu. Man ließ 30 min nachrühren, wobei sich die Mischung auf 40 °C abkühlte, und gab bei dieser Temperatur eine Mischung aus 15 RT Wasser, 0,5 RT 50 %iger Natronlauge und 3,2 Tlen. Natriumboranat zu. Man ließ 1 Stunde nachrühren und destillierte bei 2 mbar.

$Kp_2$ mbar : 133 - 135 °C
Ausbeute : 913 Tle. = 99 % d. Th.
GC-Analyse :

| | |
|---|---|
| Methylhomoveratrylamin | 99,6 % |
| Homoveratrylamin | Spur |
| Dimethylhomoveratrylamin | — |

Beispiel 2 (Hydrierung)

Es wurde analog Beispiel 1b) und c) verfahren, jedoch bei 60 statt 90 °C gearbeitet.

GC-Analyse des Rohgemisches

| | |
|---|---|
| Methylhomoveratrylamin | 91,6 % |
| Homoveratrylamin | 3,4 % |
| Leichtsiedende Bestandteile | 3,4 % |
| Destillationsrückstand | 1,6 % |

Beispiel 3 (Hydrierung)

Es wurde analog Beispiel 1b) und c) verfahren ; pro Stunde ließ man jedoch 100 RT einer 10 %igen Lösung von VCN in N-Methylpyrrolidon über den Katalysator von Beispiel 1a) strömen.

GC-Analyse des Rohgemisches :

| | |
|---|---|
| Methylhomoveratrylamin | 89,5 % |
| Homoveratrylamin | 4,1 % |
| Leichtsiedende Bestandteil | 5,1 % |
| Destillationsrückstand | 1,3 % |

Beispiel 4 (Hydrierung)

Es wurde analog Beispiel 1b) und c) verfahren, pro Stunde ließ man jedoch 75 RT einer 40 %igen Lösung von VCN in Methylpyrrolidon über den Katalysator von Beispiel 1a) strömen.

GC-Analyse des Rohgemisches :

| | |
|---|---|
| Methylhomoveratrylamin | 86,3 % |
| Homoveratrylamin | 3,1 % |
| Veratrylcyanid | 2,8 % |
| Leichtsiedende Bestandteile | 4,9 % |
| Destillationsrückstand | 2,9 % |

Beispiel 5 (Hydrierung)

Es wurde analog Beispiel 1b) und c) verfahren, pro Stunde ließ man jedoch 100 RT einer 10 %igen Lösung von VCN in Methanol über einen Katalysator aus 93,75 % Ni und 6,25 % Fe strömen.
Der Katalysator war analog zum Ni-Co-Katalysator von Beispiel 1a) hergestellt worden.

GC-Analyse des Rohgemisches :

| | |
|---|---|
| Methylhomoveratrylamin | 90,1 % |
| Homoveratrylamin | 5,3 % |
| Leichtsiedende Bestandteile | 1,9 % |
| Destillationsrückstand | 2,6 % |

Beispiel 6 (Aufarbeitung)

1 000 Tle. des gemäß Beispiel 1b) erhaltenen Gemisches wurden in einem beheizbaren Rührgefäß mit 4 000 RT Fassungsvermögen vorgelegt und auf 50 °C vorgeheizt. Dann gab man langsam unter Kühlung, um die Temperatur zu halten, 29 Tle. Benzaldehyd zu. Man ließ 30 min nachrühren, wobei sich die Mischung auf ca. 40 °C abkühlte, und gab dann bei dieser Temperatur 1 500 RT Methanol zu. Diese Mischung wurde in einen Rührautoklaven gegeben und mit 50 Teilen Raney-Nickel bei 70 °C und 100 bar hydriert, bis die $H_2$-Aufnahme vollständig war. Nach Filtration des Katalysators wurde das Methanol bei Normaldruck abdestilliert und der verbleibende Rückstand anschließend bei 2 mbar destilliert.

Ausbeute : 895 Teile = 96,7 %

GC-Analyse :

| | |
|---|---|
| Methylhomoveratrylamin | 99,2 % |
| Homoveratrylamin | 0,4 % |
| Dimethylhomoveratrylamin | — |

Vergleichsbeispiel

a) Herstellung des Katalysators gemäß DE-PS 1 493 754

959 Tle. Kieselsäurestrangpreßlinge mit einem Litergewicht von 447 g, einer Porosität von 0,96 cm³/g und einem Durchmesser der Strangpreßlinge von 4 mm wurden in einer Imprägniertrommel zunächst mit 920 RT einer Lösung bestehend aus einer Mischung von 2 355,8 Tln. einer 15,2 % Cu enthaltenden Kupfernitratlösung, 179,1 Tln. einer 6,4 % Cr enthaltenden Cr-Nitratlösung und einer Lösung von 37,23 Tln. $KNO_3$ in 150 RT VE-Wasser getränkt, bis die zugegebene Lösung vollständig aufgesaugt war. Hernach wurde noch 15 min nachrotieren lassen und die feuchten Stränge 16 h bei 120 °C getrocknet und 6 h bei 520 °C getempert.

Die Stränge hatten nach der Temperung ein Gewicht von 1 208 Tln. und eine Porosität von 0,73 $cm^3/g$. Mit dieser Menge Stränge und 880 RT weiterer Imprägnierlösung (siehe oben) wurde die oben beschriebene Prozedur wiederholt.

Nach dem zweiten Tempern wiesen die imprägnierten Strangpreßlinge ein Litergewicht von 676 g auf bei einer Porosität von 0,6 $cm^3/g$. Der fertige Katalysator hatte eine analytisch ermittelte Zusammensetzung von 32,5 % CuO, 1,2 % $Cr_2O_3$ und 1,25 % $K_2O$, alles bezogen auf den Glührückstand bei 900 °C.

b) Hydrierung

Über 250 RT des wie unter (a) beschrieben hergestellten Katalysators enthaltend 25 % Cu, 0,8 % Cr und 1 % K auf 73,2 % Kieselgel ließ man bei einer Temperatur von 140 °C stündlich 50 RT einer 20 %igen Lösung von Veratrylcyanid in N-Methylpyrrolidon und 162 Tle. Monomethylamin bei einem Druck von 200 bar in Gegenwart von Wasserstoff (ca. 120 000 RT pro Stunde drucklos gemessen) strömen. Die Aufarbeitung erfolgte wie bei Beispiel 1.

| | |
|---|---|
| Methylhomoveratrylamin | 55,2 % |
| Homoveratrylamin | 9,8 % |
| Veratrylcyanid | 22,4 % |
| Leichtsiedende Bestandteile | 5,1 % |
| Destillationsrückstand | 7,5 % |

c) Es wurde wie unter b) verfahren, die Reaktionstemperatur betrug jedoch 180 °C.

| | |
|---|---|
| Methylhomoveratrylamin | 70,1 % |
| Homoveratrylamin | 10,3 % |
| Veratrylcyanid | — |
| Leichtsiedende Bestandteile | 8,8 % |
| Destillationsrückstand | 10,8 % |

**Patentansprüche**

1. Verfahren zur Herstellung von Methylhomoveratrylamin durch katalytische Hydrierung von Veratrylcyanid (VCN) in Gegenwart von Methylamin, dadurch gekennzeichnet, daß man VCN im Molverhältnis Methylamin zu VCN von mindestens 10 : 1 in Gegenwart eines Nickel-Katalysators mit mindestens 70 Gew.% Nickel bei 20 bis 140 °C und einem Wasserstoffdruck von 100 bis 300 bar hydriert und das erhaltene Rohprodukt, das geringe Mengen Homoveratrylamin enthält, mit zu diesem etwa

äquimolaren Mengen eines Aldehyds versetzt, der in alpha-Stellung keinen Wasserstoff enthält, die entstandene Schiff'sche Base hydriert und das Methylhomoveratrylamin aus diesem Gemisch destilliert.

2. Kontinuierliches Verfahren nach Anspruch 1, dadurch gekennzeichnet, daß man das bei der Reaktion entstehende Ammoniak von dem im Überschuß verwendeten Monomethylamin abtrennt und das Monomethylamin der Reaktion wieder zuführt.

**Claims**

1. A process for the preparation of methyl homoveratrylamine by catalytic hydrogenation of veratryl cyanide (VCN) in the presence of methylamine, wherein VCN is hydrogenated in the presence of a nickel catalyst containing at least 70 % by weight of nickel at 20 to 140 °C and under a hydrogen pressure of from 100 to 300 bars, the molar ratio of methylamine to VCN being at least 10 : 1 ; an approximately equimolar amount of an aldehyde which does not contain hydrogen in the alpha-position is added to the resulting crude product which contains small amounts of homoveratrylamine ; the resulting Schiff base is hydrogenated ; and the methyl homoveratrylamine is distilled from this mixture.

2. A continuous process as claimed in claim 1, wherein the ammonia formed in the reaction is separated from the monomethylamine used in excess, and the monomethylamine is returned to the reaction.

**Revendications**

1. Procédé de préparation de méthylhomoveratrylamine par hydrogénation catalytique de cyanure de veratryle (VCN) en présence de méthylamine, caractérisé par le fait qu'on hydrogène du VCN, dans un rapport molaire, méthylamine à VCN, d'au moins 10/1, en présence d'un catalyseur de nickel, avec au moins 70 % en poids de nickel, à une température de 20 à 140 °C et une pression d'hydrogène de 100 à 300 bar et l'on additionne le produit obtenu, qui contient de faibles quantités de homoveratrylamine, avec des quantités sensiblement équimolaires à celles-ci d'un aldéhyde qui ne contient pas d'hydrogène en position alpha, on hydrogène la base de Schiff créée et on fait distiller la méthylhomoveratrylamine de ce mélange.

2. Procédé en continu selon la revendication 1, caractérisé par le fait qu'on sépare l'ammoniac produit lors de la réaction, de la monométhylamine utilisée en excès et l'on ramène la monométhylamine à la réaction.